# EUROPÄISCHE PATENTSCHRIFT

(11) **EP 2 268 392 B1**
(45) Veröffentlichungstag und Bekanntmachung des Hinweises auf die Patenterteilung: **02.11.2011**
(21) Anmeldenummer: 09732211.9
(22) Anmeldetag: 09.04.2009
(51) Int. Cl.: B01J 20/00, G01N 30/00, C07K 1/16

(54) **VERFAHREN ZUR ISOLIERUNG VON POLYPEPTIDEN**
METHOD FOR ISOLATING POLYPEPTIDES
PROCÉDÉ D'ISOLEMENT DE POLYPEPTIDES

(30) Priorität: 16.04.2008 DE 102008019338
(43) Veröffentlichungstag der Anmeldung: 05.01.2011
(73) Patentinhaber: Qiagen GmbH, 40724 Hilden (DE)
(72) Erfinder: FECKLER, Christian, 52076 Aachen (DE)
(74) Vertreter: Roth, Carla
(86) Internationale Anmeldenummer: PCT/EP2009/002637
(87) Internationale Veröffentlichungsnummer: WO 2009/127359

(56) Entgegenhaltungen:
- DE-B4- 19 926 056
- TOMMY NORDSTRÖM ET AL: "Generation of a new protein purification matrix by loading ceramic hydroxyapatite with metal ions-demonstration with poly-histidine tagged green fluorescent protein" JOURNAL OF BIOTECHNOLOGY, ELSEVIER SCIENCE PUBLISHERS, AMSTERDAM, NL, Bd. 69, 1. Januar 1999 (1999-01-01), Seiten 125-133, XP002464877 ISSN: 0168-1656
- ALDINGTON ET AL: "Scale-up of monoclonal antibody purification processes" JOURNAL OF CHROMATOGRAPHY B: BIOMEDICAL SCIENCES & APPLICATIONS, ELSEVIER, AMSTERDAM, NL, Bd. 848, Nr. 1, 12. März 2007 (2007-03-12), Seiten 64-78, XP005922828 ISSN: 1570-0232

## Beschreibung

Die vorliegende Erfindung betrifft ein Verfahren zur Aufreinigung von Polypeptiden durch die Nutzung von Borcarbid-Trägermaterialien.

Die Aufreinigung von Proteinen aus heterogenen Mischungen ist oftmals ein Mehrschritt-Prozess unter Nutzung der physikalischen, chemischen und elektrischen Eigenschaften der aufzureinigenden Proteine. Entscheidende Charakteristika eines Proteins, die relevant für die Aufreinigung sind, sind die Löslichkeit, die Ladung, die Größe und die spezifische Bindungskapazität. Die Isolierung und Reinigung von Proteinen ist daher eine besondere Herausforderung, aufgrund der unterschiedlichen chemischen und physikalischen Eigenschaften dieser Biomoleküle. Außerdem sind die Materialen, aus denen die Proteine isoliert werden, sowie die nachfolgenden Anwendungen der isolierten Proteine vielfältig. Daher ist es notwendig, die bereits existierenden Methoden zur Aufreinigung und Isolation von Proteinen zu erweitern.

Ein gängiges Prinzip zur Isolation von Polypeptiden/Proteinen ist die Festphasen-Extraktion (SPE). Dabei wird der Analyt (Polypeptid/Protein) an einen festen Träger gebunden und nach dem Waschen eluiert. Ein Nachteil bestehender SPE-Systeme ist die Bindung unter stark sauren Bedingungen (beispielsweise pH < 1,5) und die Elution mit organischen Lösungsmitteln wie Acetonitril (ACN). Diese Bedingungen können denaturierend auf die Proteine wirken und damit die Anwendungs- und Analysemöglichkeiten der isolierten Proteine verschlechtern. Zudem ist ACN ein als gefährlich einzustufendes Lösungsmittel, das auch für viele sich anschließende Analysetechniken entfernt werden muss.

Neben den gängigen SPE-Produkten die auf Umkehrphasen-Chemie (reversed phase) wie z.B. C18 Materialien beruhen, gibt es auch SPE-Systeme die auf Siliziumcarbid-Keramik Basis bestehen (siehe bspw. WO 03/044043). Die Proteinbindung an die feste Phase wird hier ebenfalls durch Ansäuerung, also Protonierung der Proteine erzielt.

Der vorliegenden Erfindung liegt daher die Aufgabe zugrunde, geeignete Verfahren sowie geeignete Festphasen zur Isolierung, insbesondere Aufreinigung und/oder Entfernung von Polypeptiden aus einer Probe zur Verfügung zu stellen.

Erfindungsgemäß wird ein Verfahren zur Isolation und/oder Aufreinigung von wenigstens einem Polypeptid aus einer polypeptidhaltigen Probe zur Verfügung gestellt, das dadurch gekennzeichnet ist, dass die polypeptidhaltige Probe mit einem Borcarbid-Trägermaterial bei einem pH-Wert in Kontakt gebracht wird, der die Bindung des Polypeptids an das Borcarbid-Trägermaterial erlaubt.

Es wurde überraschend festgestellt, dass Borcarbid besonders gute Polypeptid-Bindungseigenschaften aufweist. So können, wie die Beispiele zeigen, sowohl Peptide als auch Proteine gebunden werden; darüber hinaus lassen sich auch phosphorylierte Polypeptide effektiv binden und damit isolieren, aufreinigen und/oder aufkonzentrieren.

Das erfindungsgemäße Verfahren kann zum einen dazu dienen, Polypeptide aus einer Probe zu entfernen. Die gezielte Entfernung von Polypeptiden kann beispielsweise vorteilhaft sein, um andere Biomoleküle wie z.B. Nukleinsäuren oder kleinere organische Moleküle wie z. B. Arzneiwirkstoffe oder Metaboliten ohne störende Polypeptidverunreinigungen leichter analysieren bzw. untersuchen zu können. Das erfindungsgemäße Verfahren zur Isolation von wenigstens einem Polypeptid umfasst daher auch Ausführungsformen, bei denen Polypeptide aus einer Probe entfernt werden. Die aus der Probe zu entfernenden Polypeptide binden an das Borcarbid-Trägermaterial und sind damit nicht oder in geringerer Menge im Durchbruch (im Falle des Einsatzes einer Borcarbidsäule) oder im Überstand (bspw. beim Einsatz von magnetischen Partikeln mit einer Borcarbid-Oberfläche) enthalten. Sofern gewünscht, kann auch in diesem Fall eine Elution der Polypeptide von dem Borcarbid-Trägermaterial erfolgen, um die Polypeptidfraktion getrennt von der übrigen Probe weiter zu verarbeiten, beispielsweise zu analysieren.

Der Einsatz von Borcarbid als Trägermaterial zur Bindung von Polypeptiden funktioniert im wesentlich nach dem Prinzip der Festphasen-Extraktion (SPE). Die Polypeptide adsorbieren an das Borcarbid-Trägermaterial, vermutlich durch ionische Interaktionen.

Vorzugsweise erfolgt die Bindung der Polypeptide an das Borcarbid-Trägermaterial bei einem pH-Wert, der unter dem pl-Wert des zu bindenden Polypeptides (bzw. der zu bindenden Polypeptide) liegt. Vorzugsweise erfolgt die Bindung bei einem pH-Wert, der wenigstens 0,5 pH Einheiten unter dem pl liegt. Der isoelektrische Punkt (pl) des zu isolierenden Polypeptids kann durch bekannte Methoden wie beispielsweise isoelektrische Fokussierung in einem Polyacrylamidgel bestimmt werden. Der isoelektrische Punkt kann jedoch auch annähernd durch prädiktive Methoden bestimmt werden, beispielsweise durch die Analyse der Aminosäurezusammensetzung und der Berechnung der Ladungen der ionisierbaren Gruppen bei verschiedenen pH-Werten unter Nutzung der Henderson-Hasselbach Gleichung.

Vorzugsweise erfolgt die Bindung bei einem sauren pH-Wert. Bei einem sauren pH-Wert können insbesondere viele verschiedene Polypeptide effektiv gebunden und damit isoliert werden. Der saure pH-Wert bei der Bindung kann beispielsweise dadurch eingestellt werden, dass säurehaltige Lösungen oder saure Puffer zu der polypeptidhaltigen Probe gegeben werden, um sie auf den gewünschten pH-Wert anzusäuern.

Gemäß einer weiteren Ausführungsform wird das Borcarbid-Trägermaterial vor dem in Kontakt bringen mit der polypeptidhaltigen Probe bei einem sauren pH-Wert äquilibriert. Durch die Äquilibrierung wird das Borcarbid-Trägermaterial optimal auf die Bindung der Polypeptide vorbereitet.

Vorzugsweise ist der pH-Wert bei der Bindung in der Probengemisch ≤ 6, ≤ 5, ≤ 4,5, ≤ 4 oder sogar ≤ 3,5. Vorzugsweise liegt der pH-Wert jedoch oberhalb von 1,5 und besonders bevorzugt oberhalb von 2,5, um zu stark saure Bedingungen, die denaturierend auf die Polypeptide wirken könnten, zu vermeiden.

Entsprechende saure Bedingungen werden vorzugsweise während der Bindung der Polypeptide an das Borcarbid-Trägermaterial in dem polypeptidhaltigen Probengemisch sowie zur Äquilibierung des Borcarbid-Trägermaterials (sofern durchgeführt) eingestellt.

Demgegenüber erfolgt die Elution des an das Borcarbid-Trägermaterial gebundenen Polypeptides bzw. Polypeptide vorzugsweise bei einem pH-Wert, der über dem pl des zu eluierenden Polypeptides liegt. Vorzugsweise liegt der pH-Wert eine pH Einheit über dem isoelektrischen Punkt (pl).

Vorzugsweise ist der pH-Wert bei der Elution alkalisch und liegt bei ≥9, ≥ 10 und besonders bevorzugt ≥ 11. Bei einem pH-Wert ≥ 11 wurden besonders gute Ergebnisse bei der Elution von dem erfindungsgemäßen Borcarbid-Trägermaterial erzielt.

Zur Einstellung des pH-Wertes können, wie ausgeführt, säure- oder basehaltige Lösungen eingesetzt werden. Darüber hinaus können auch saure oder basische Puffer als Lösungen eingesetzt werden. Beispiele für saure Puffer sind z. B. Acetatpuffer, Citratpuffer oder Glycinpuffer. Gängige basische Puffer sind beispielsweise Trispuffer, Bicinepuffer, TAPS-Puffer oder Tricinepuffer.

Der Begriff "Polypeptid" bezieht sich auf ein Polymer von Aminosäuren, die über Peptidbindungen verknüpft sind. Der Begriff "Polypeptid" umfasst Polypeptide jeglicher Länge, einschließlich Proteine (mit einer Länge von mehr als 50 Aminosäuren) sowie Peptide (mit einer Länge von 2 bis 49 Aminosäuren). Erfindungsgemäß können ein oder mehrere Polypeptide aus einer Probe isoliert, aufgereinigt, aufkonzentriert und/oder entfernt werden.

Die Probe, aus der die Polypeptide isoliert werden, kann jeglicher Art sein. Beispielsweise genannt seien polypeptidhaltige Lösungen, Körperflüssigkeiten wie Plasma, Urin, Sperma, Tränen, Speichel und Cerebralflüssigkeit. Darüber hinaus lassen sich auch Polypeptide aus zellhaltigen Proben, wie beispielsweise Zelllysaten oder polypeptidhaltigen Kulturmedien isolieren. Besonders problematisch ist die Isolierung von Polypeptiden aus verdünnten und insbesondere salzhaltigen Proben wie beispielsweise Urin. Bei solchen Lösungen/Proben liefert das erfindungsgemäße Verfahren besonders gute Ergebnisse.

Neben dem erfindungsgemäßen Verfahren zur Isolation von Polypeptiden aus polypeptidhaltigen Proben wird ferner eine Matrix zur Bindung von Polypeptiden zur Verfügung gestellt, welche dadurch gekennzeichnet ist, dass sie Borcarbid aufweist. Die Matrix kann letztlich in vielfältigen Formen vorliegen, so beispielsweise als Säule, Membran, Beads, insbesondere magnetische Beads, sowie als Chipoberfläche. Letztlich entscheidend ist, dass die Matrix eine Borcarbid-haltige Ober- bzw. Adsorptionsfläche aufweist, die für die Bindung der Polypeptide aus der Probe zugänglich ist.

Die erfindungsgemäße Matrix kann bspw. in Form eines Säulenkörpers vorliegen. Vorzugsweise weist der Säulenkörper einen Hohlraum zur Aufnahme des Borcarbid-Trägermaterials auf. Es hat sich gezeigt, dass auch die Geometrie eines solchen Säulenkörpers entscheidend für die Effektivität der Polypeptidbindung aus der Probe sein kann. Dies insbesondere, wenn die Probe eine Vielzahl und insbesondere auch unterschiedliche Polypeptide aufweist. In einem solchen Fall ist eine möglichst effektive Bindung der Polypeptide an das Borcarbid-Trägermaterial notwendig, um die Polypeptide umfassend aus der Probe zu entfernen. Hier hat es sich gezeigt, dass es besonders vorteilhaft ist, den Querschnitt der Borcarbid-Trägermatrix möglichst gering und den Kontaktweg der Probe mit dem Borcarbid-Trägermaterial möglichst lang zu gestalten. Aus diesem Grunde ist der Säulenkörper vorzugsweise deutlich länger als breit. Der das Borcarbid-Trägermaterial aufnehmende Hohlraum des Säulenkörpers ist mindestens doppelt so lang, vorzugsweise 3, 4, 5 oder wenigstens 6 mal so lang wie breit. Durch diese Ausgestaltung bleibt die Probe beim Durchlaufen des Borcarbid-Trägermaterials möglichst lange mit dem Trägermaterial über eine lange Strecke hin in Kontakt, wodurch die Polypeptide besonders effektiv gebunden werden. Wie die Beispiele belegen, führt eine entsprechende Säulengeometrie zu deutlich verbesserten Ergebnissen. Es werden mehr Polypeptide aus der Probe gebunden, so dass sich weniger Polypeptide im Durchbruch befinden.

Mit der Erfindung wird ferner ein Verfahren zur Herstellung eines Säulenkörpers zur Verfügung gestellt, welches dadurch gekennzeichnet ist, dass ein Säulenkörper mit einem Hohlraum der oben beschriebenen Geometrie mit einem Borcarbid-Trägermaterial beladen wird. Die Beladung kann beispielsweise dadurch erfolgen, dass das trockene Borcarbid-Pulver in den Hohlraum eingeführt wird. Durch Verwendung eines Stempels kann das Borcarbid-Trägermaterial dicht gepackt werden.

Ferner betrifft die vorliegende Erfindung die Verwendung eines Borcarbid-Trägermaterials oder einer Matrix wie oben beschrieben zur Bindung von Polypeptiden.

Ferner wird ein Kit zur Isolation von Polypeptiden aus einer polypeptidhaltigen Probe zur Verfügung gestellt, welcher dadurch gekennzeichnet ist, dass er eine borcarbidhaltige Matrix aufweist. Einzelheiten der borcarbidhaltigen Matrix sind oben im Detail beschrieben; wir verweisen auf unsere obigen Ausführungen.

Der erfindungsgemäße Kit kann ferner einen oder mehrere der folgenden Bestandteile aufweisen:
- einen sauren Bindungs- und/oder Aquilibrierungspuffer,
- einen bevorzugt alkalischen Elutionspuffer

Das erfindungsgemäße Verfahren kann bspw. durchgeführt werden, indem man eine Aufschwemmung der polypeptidhaltigen Probe (ggf. durch Lyse aufgeschlossen) und dem Borcarbid-Trägermaterial bei dem oben beschriebenen Bindungs-pH erzeugt. Das Borcarbid kann bspw. aus der Mischung entfernt werden, nachdem das Polypeptid gebunden wird. Alternativ können nicht gebundene Bestandteile durch Zentrifugation entfernt werden. Die Elution kann bspw. durch die Herstellung einer Aufschwemmung des Borcarbids mit einer geeigneten Elutionslösung erfolgen. Auch kann ein Elutionspuffer eingesetzt werden. Geeignete Elutions-Bedingungen sind oben im Detail beschrieben.

Alternativ hierzu kann das Borcarbid in einen Säulenkörper eingeführt werden und auf den gewünschten Bindungs-pH eingestellt bzw. äquilibiert werden. Die polypeptidhaltige Probe, die auf den gewünschten Bindungs-pH-Wert eingestellt wird, wird in herkömmlicher Weise über die Säule gegeben. Nach der Polypeptid bindung erfolgt die Elution mit der gewünschten Elutionslösung bzw. bei dem gewünschten Elutions-pH.

Gemäß einer weiteren Ausführungsform kann das Borcarbid vorzugsweise auf den gewünschten Bindungs-pH-Wert äquilibriert, in eine Zentrifugationsäule (spin column) gegeben werden. Die polypeptidhaltige Probe, die auf den gewünschten Binde-pH-Wert eingestellt wird, wird auf die Oberfläche des Borcarbid-Trägermaterials gegeben. Die Säule wird zentrifugiert, um die wässrige Phase zu separieren; die wässrige Phase wird verworfen. Das an das Borcarbid-Trägermaterial gebundene Protein wird gewaschen und bei dem gewünschten pH-Wert eluiert. Dies erfolgt durch Zugabe der gewünschten Elutionslösung, um den Elutions-pH-Wert einzustellen, gefolgt von einem Zentrifugationsschritt, um die wässrige Phase enthaltende das isolierte Protein zu eluieren.

Der Einsatz von Zentrifugationssäulen ist bei Einsatz des erfindungsgemäßen Borcarbid-Trägermaterials unproblematisch, da das Borcarbid Zentrifugationskräften gut widersteht.

### Beschreibung der Figuren

### Figur 1a und 1b:

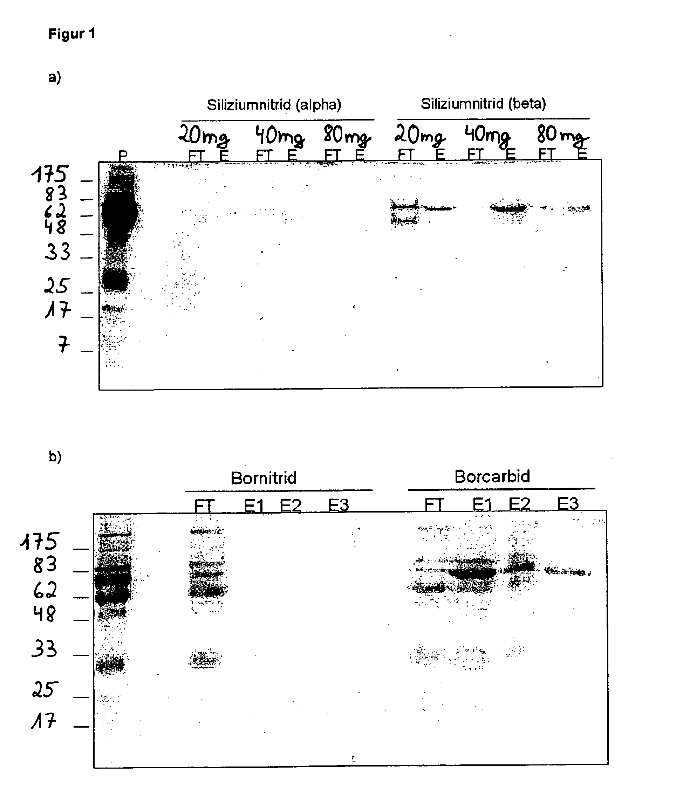

Gezeigt ist die Polypeptidbindungskapazität verschiedener potentieller Trägermaterialien (Siliziumnitrit (alpha), Siliziumnitrid (beta), Bornitrid und Borcarbid). Gezeigt sind Ergebnisse der Gelelektrophorese. Verwendet wurde ein 12 % Polyacrylamid Gel nach Lämmli; gefärbt wurde mit Coomassie brilliant blau. Wie insbesondere Figur 1b zeigt, ist nur Borcarbid in der Lage, Polypeptide effektiv zu binden und damit aus einer polypeptidhaltigen Probe zu entfernen, bzw. die Polypeptide bei der Elution wieder abzugeben.
FT: Durchbruch Spin-Säule
E: Eluatfraktion

### Figur 2:

Gezeigt ist der Einfluss der Säulengeometrie auf das Aufreinigungsverhalten des Borcarbid-Trägermaterials. Mit der getesteten Säule mit Inlay (wodurch eine Säulengeometrie erzielt wurde, bei der die Länge der Borcarbidsäule ca. 5 mal so lang wie breit war konnten deutlich bessere Bindungs- und Elutionsergebnisse erzielen als mit einer Säule ohne Inlay.
FT: Durchbruch Spin-Säule
E: Eluatfraktionen

### Figur 3:

Gezeigt ist die Säulengeometrie gemäß einer vorteilhaften Ausführungsform. Bevorzugt weist der äußere Umlauf des Säulenkörpers (1) eine trichterförmige Verjüngung (2) auf, um das Borcarbid-Trägermaterial einfacher in den Hohlraum (Inlay) (3) einführen zu können. In die gezeigte Ausführungsform passen ca. 50 bis 60 mg Borcarbid. Dieses wird zentrifugiert, um es in eine kompakte Form zu bringen und entsprechend zusammenzupressen.

### Figur 4a und 4b:

Gezeigt sind die Ergebnisse der Bindung von Proteinen an das Borcarbid-Trägermaterial aus Plasma- und Urinproben. Gezeigt sind die Ergebnisse der Gelelektrophorese. Figur a zeigt die Ergebnisse einer Coomassifärbung bei der Aufreinigung von Polypeptiden aus Plasma. Figur b zeigt die Ergebnisse der Polypeptidaufreinigung aus einer Urinprobe anhand einer Silberfärbung. Die Silberfärbung zeichnet sich durch ihre hohe Sensitivität im Vergleich zu Coomassigefärbten Gelen aus.
M: Proteinstandard
P: Plasma
ST: Durchbruch
E: Eluatfraktionen

### Figur 5a und 5b:

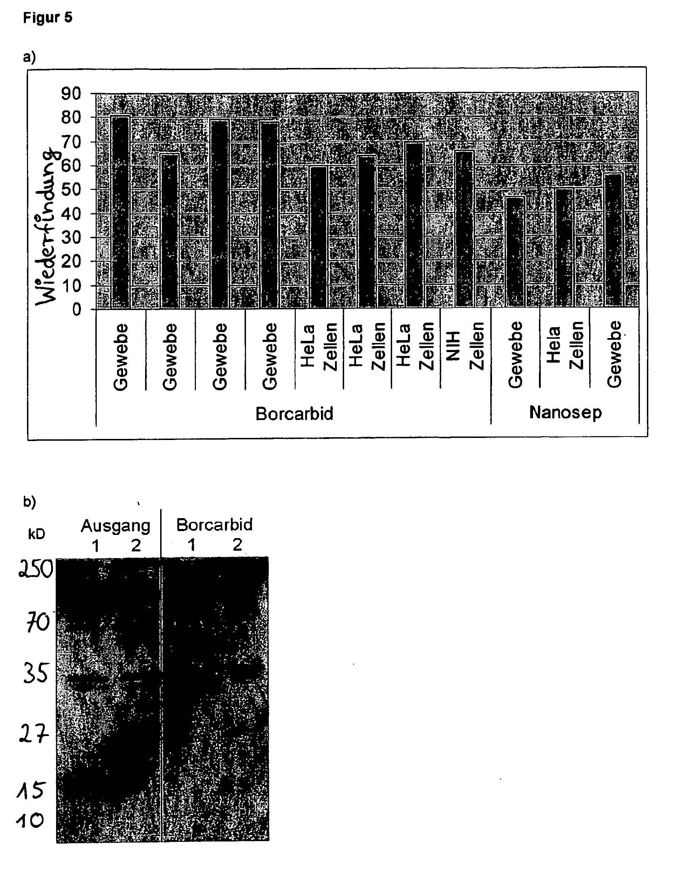

Figur 5a zeigt die Ausbeute an phosphorylierten Polypeptiden (100 µg) aus verschiedenen Proben. Zunächst wurden mit Hilfe einer Affinitätssäule (PhosphoProteinPurification Kit, QIAGEN) phosphorylierte Proteine isoliert und dann das Eluat konzentriert. Verglichen wurde das erfindungsgemäße Verfahren unter Einsatz von Borcarbid mit einem Verfahren gemäß dem Stand der Technik (Nanosep). Figur 5b zeigt die Ergebnisse eines Western Blots. Pro Spur wurden 10 µg Protein aufgetragen. Im Ausgang befand sich das Eluat der PhosphoProtein Purification Säule konzentriert mit Nanosep Ultrafiltrationssäulen. Der Versuch wurde in einer Doppelbestimmung durchgeführt.

### Figur 6a und 6b:

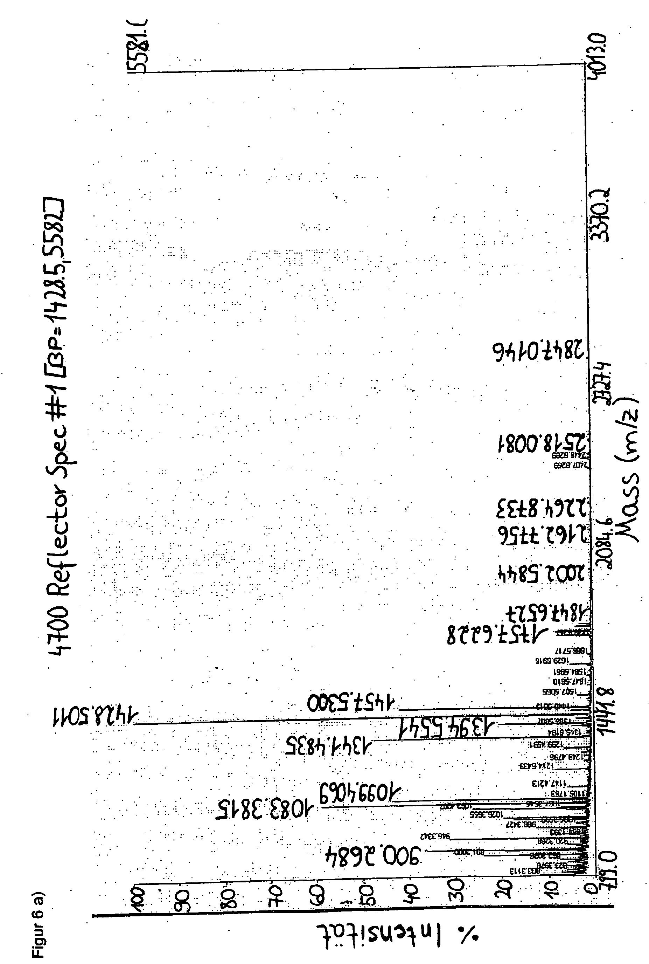

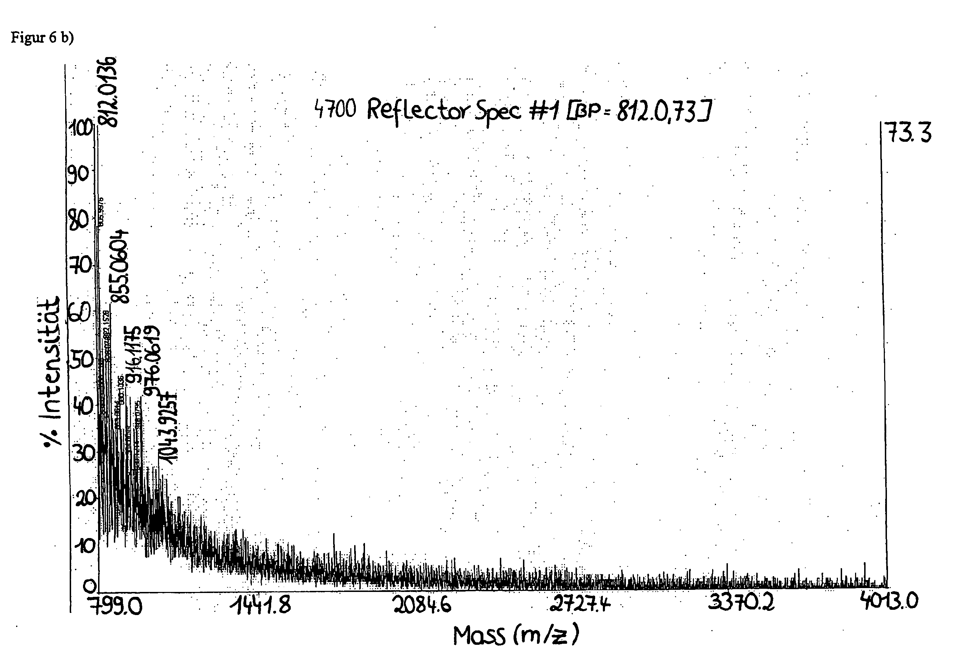

Gezeigt sind die Ergebnisse der Isolierung von Peptiden (hier von beta-Galaktosidase) mit dem erfindungsgemäßen Verfahren. Figur 6a zeigt die MALDI-TOF-MS Ergebnisse im Eluat der Borcarbidsäule. Figur b zeigt die vorhandenen Peptide im Durchbruch der Säule (keine signifikanten Mengen).

### Beispiele

Die Erfindung wird nunmehr anhand von illustrativen Beispielen erläutert. Diese dienen ausschließlich dazu, die Erfindung näher zu erläutern; sie sind nicht beschränkend.

### Beispiel 1: Protokoll zur Durchführung des erfindungsgemäßen Verfahrens

Wenn nicht anders angegeben wurden 60 mg Borcarbid oder andere Keramiken eingewogen und in Spinsäulen gefüllt. Die Säulen wurden mit 50 mM Glycin pH3,5 äquilibriert. Die Proteinlösung wurde mit 1 M Glycin pH 1,5 auf einen pH-Wert von 3,5 eingestellt und auf die Spin-Säule gegeben. Entweder wurde für 30 min durch Mischen inkubiert oder bei Verwendung des Inlays direkt zentrifugiert.

Das Waschen der gebundenen Polypeptide erfolgte mit 50 mM Glycin pH 3,5. Eluiert wurde mit 10 mM Tris pH 12.

### Beispiel 2: Borcarbid im Vergleich zu anderen Keramiken

Vier verschiedene Keramiken (Siliziumnitrid (alpha), Siliziumnitrid (beta), Bornitrid und Borcarbid) wurden auf ihre Polypeptid-Bindungskapazität hin untersucht. Plasma wurde als polypeptidhaltige Probe eingesetzt; vorgegangen wurde nach dem Protokoll des Bsp. 1. Nur Borcarbid zeigt eine Bindung der Plasmaproteine mit erfolgreicher Elution.
Fig. 1 zeigt die Ergebnisse.

### Beispiel 2: Einfluss der Geometrie des Säulenkörpers (komprimiertes Pulver)

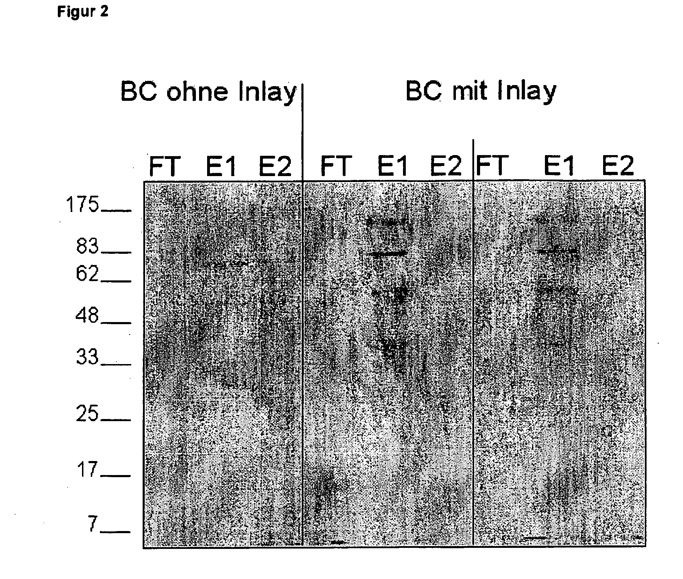

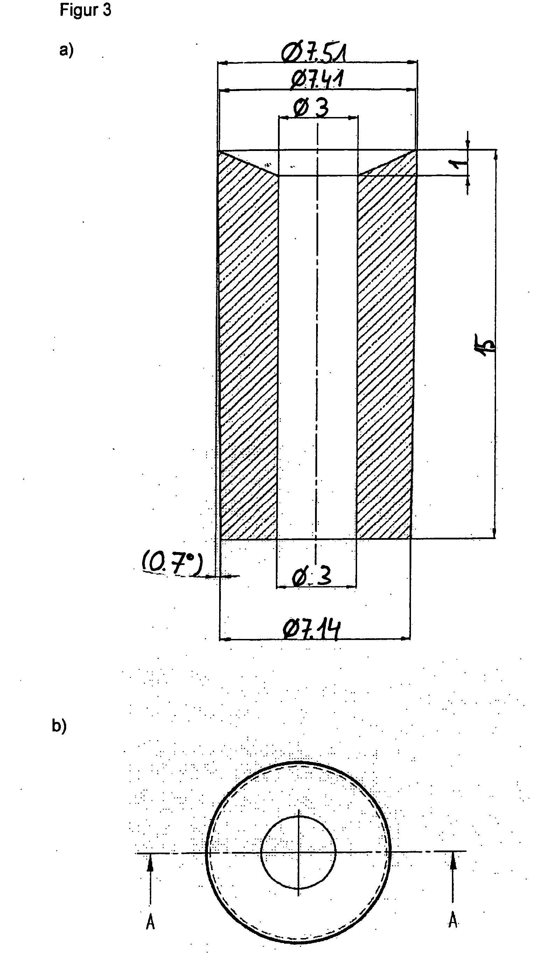

Während der Tests wurde festgestellt, dass durch Veränderung der Säulengeometrie (Verkleinerung des Säuleninnendurchmessers) eine verbesserte Wiederfindung und damit Ausbeute der Proteine erzielt werden konnte (siehe Fig. 2). Um die Verkleinerung des Säuleninnendurchmessers vorzunehmen, wurde ein Plastikröhrchen entworfen, das in die bestehenden Säulenkörper (DyeEx-Spin-Säule) eingesetzt wurde (Fig. 3). Das Keramikpulver wurde in dieses Innenröhrchen gefüllt und komprimiert. Dadurch wird die Probe über die gesamte/verlängerte Strecke des langgestreckten Säulenkörpers geführt, wodurch die Bindung der Polypeptide deutlich verbessert wird.

### Beispiel 3: Einsatz verschiedener polypeptidhaltiger Probenmaterialien

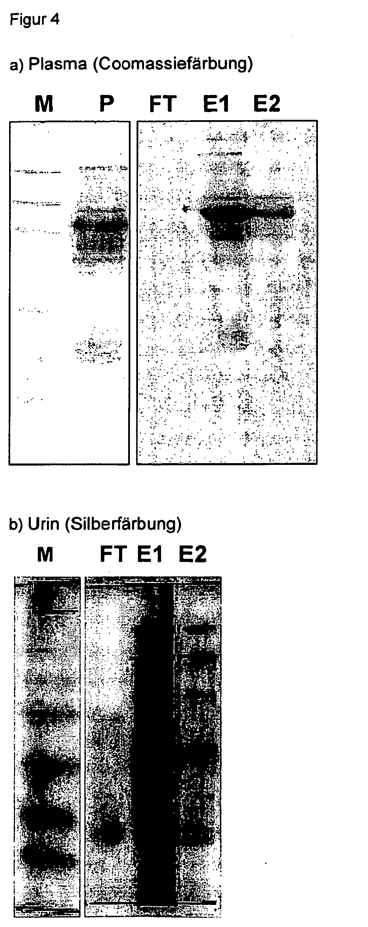

Die in Fig. 4 gezeigten Anwendungen belegen, dass mit dem erfindungsgemäßen Verfahren Proteine gezielt aus unterschiedlichen polypeptidhaltigen Proben isoliert werden können. In A) wurde verdünntes Plasma verwendet in B) 1 ml Urin.

### Beispiel 4: Isolierung von phosphorylierten Proteinen

Nach der Isolierung von phosphorylierten Proteinen mit dem PhosphoProteinPurification Kit (QIAGEN) wurden je 100 µg der Eluate über eine Borcarbidsäule (mit etwa 60 mg Pulver) gebunden und eluiert. Die Ausbeute beträgt etwa 60-80 %. Im Vergleich dazu wurde die Proteinlösung mit einer gängigen Ultrafiltrationssäule konzentriert. Die Wiederfindung beträgt dabei < 60 % (Fig. 5 A).

Im Western-Blot Experiment (Fig. 5 B) wurde die Identität der phosphorylierten Proteine bestätigt. Als Antikörper wurde ein anti-phospho-Tyrosin-Antikörper verwendet (4G10).

Vorliegend wurde der PhosphoKit vorab eingesetzt, um die besondere Effezienz des Verfahrens bei der Aufreinigung von Phosphoproteinen zu demonstrieren. Eine entsprechende Effizienz wird auch bei der Aufreinigung aus einer gemischten Probe (phosphorylierte und nicht-phosphorylierte Proteine bzw. Peptide) erzielt.

### Beispiel 5: Isolierung von Peptiden

Um zu überprüfen, ob das erfindungemäße Verfahren auch zur Isolierung von Peptiden verwendet werden kann, wurde eine Peptidmischung (tryptische Fragmente der β-Galaktosidase) prozessiert. Durch MALDI-TOF-MS konnte nachgewiesen werden, daß bis auf ein Peptid alle Peptide der Mischung gebunden und eluiert werden konnten. Im Durchbruch konnten keine signifikanten Mengen Peptid nachgewiesen werden. Das Verfahren ist daher auch sehr gut geeignet, um kleinere Peptide aus einer Probe zu isolieren.

## Patentansprüche

1. Verfahren zur Isolation und/oder Aufreinigung von wenigstens einem Polypeptid aus einer polypeptidhaltigen Probe, **dadurch gekennzeichnet, dass** die Probe mit einem Borcarbid-Trägermaterial bei einem pH-Wert in Kontakt gebracht wird, der die Bindung des Polypeptids an das Borcarbid-Trägermaterial erlaubt.

2. Verfahren nach Anspruch 1, **dadurch gekennzeichnet, dass** die Isolation des wenigstens einen Polypeptides erfolgt, um dieses aus der Probe zu entfernen.

3. Verfahren nach Anspruch 1 oder 2, **dadurch gekennzeichnet, dass** das wenigstens ein Polypeptid von dem Borcarbid-Trägermaterial eluiert wird.

4. Verfahren nach einem der Ansprüche 1 bis 3, **dadurch gekennzeichnet, dass** die Bindung bei einem pH-Wert erfolgt, der unter dem pl des zu bindenden Polypeptids liegt.

5. Verfahren nach einem oder mehreren der Ansprüche 1 bis 4, **dadurch gekennzeichnet, dass** die Bindung bei einem sauren pH- Wert erfolgt.

6. Verfahren nach einem oder mehreren der Ansprüche 1 bis 5, **dadurch gekennzeichnet, dass** das Borcarbid-Trägermaterial bei einem sauren pH-Wert äquilibriert wird.

7. Verfahren nach Anspruch 5 oder 6, **dadurch gekennzeichnet, dass** der pH-Wert ≤ 6, ≤ 5, ≤ 4,5, ≤ 4 oder ≤ 3,5 ist.

8. Verfahren nach einem oder mehreren der Ansprüche 1 bis 7, **dadurch gekennzeichnet, dass** die Elution des wenigstens einen Polypeptids bei einem pH-Wert erfolgt, der über dem pl des zu eluierenden Polypeptids liegt.

9. Verfahren nach Anspruch 8, **dadurch gekennzeichnet, dass** der pH-Wert ≥ 9, ≥ 10, oder ≥ 11 ist.

10. Verfahren nach einem oder mehreren der Ansprüche 1 bis 9, **dadurch gekennzeichnet, dass** die Probe ausgewählt ist aus der Gruppe der salzhaltigen Lösungen, Körperflüssigkeiten, Plasma, Urin, Zelllysate, Sperma, Tränen, Schweiß, Speichel und Cerebralflüssigkeit.

11. Matrix zur Bindung von Polypeptiden, **dadurch gekennzeichnet, dass** diese Borcarbid aufweist und die Matrix ein Säulenkörper ist, wobei der Säulenkörper einen Hohlraum zur Aufnahme des Borcarbid-Trägermaterials aufweist, und wobei der Hohlraum des Säulenkörpers wenigstens doppelt so lang, vorzugsweise 3, 4, 5 oder 6 mal so lang wie breit ist.

12. Verfahren zur Herstellung eines Säulenkörpers nach Anspruch 11, **dadurch gekennzeichnet, dass** ein Säulenkörper gemäß Anspruch 11 mit einem Borcarbid-Trägermaterial beladen wird.

13. Verwendung eines Borcarbid-Trägermaterials, einer Matrix, die Borcarbid aufweist oder einer Matrix nach Anspruch 11 zur Bindung von Polypeptiden.

14. Kit zur Aufreinigung und/oder Isolation von Polypeptiden, **dadurch gekennzeichnet, dass** er eine Borcarbid aufweisende Matrix oder eine Matrix gemäß Anspruch 11 aufweist.

15. Kit gemäß Anspruch 14, **dadurch gekennzeichnet, dass** dieser ein oder mehrere der folgenden Bestandteile aufweist:
- einen sauren Bindungs- und/oder Äquillibrierungspuffer und/oder
- einen alkalischen Elutionspuffer.

## Claims

1. Method for isolating and/or purifying at least one polypeptide from a polypeptide-containing sample, **characterized in that** the sample is contacted with a boron carbide support material at a pH which allows the binding of the polypeptide to the boron carbide support material.

2. Method according to Claim 1, **characterized in that** the at least one polypeptide is isolated in order to remove it from the sample.

3. Method according to either Claim 1 or 2, **characterized in that** the at least one polypeptide is eluted from the boron carbide support material.

4. Method according to any one of Claims 1 to 3, **characterized In that** binding takes place at a pH which is below the pl of the polypeptide to be bound.

5. Method according to one or more of Claims 1 to 4, **characterized In that** binding takes place at an acidic pH.

6. Method according to one or more of Claims 1 to 5, **characterized in that** the boron carbide support material is equilibrated at an acidic pH.

7. Method according to either Claim 5 or 6, **characterized in that** the pH is ≤6, ≤5, ≤4.5, ≤4, or ≤3.5.

8. Method according to one or more of Claims 1 to 7, **characterized in that** the at least one polypeptide is eluted at a pH which is above the pl of the polypeptide to be eluted.

9. Method according to Claim 8, **characterized in that** the pH is ≥9, ≥10, or ≥11.

10. Method according to one or more of Claims 1 to 9, **characterized in that** the sample is selected from the group consisting of salt-containing solutions, body fluids, plasma, urine, cell lysates, semen, teardrops, sweat, saliva, and cerebrospinal fluid.

11. Matrix for binding polypeptides, **characterized in that** it comprises boron carbide and the matrix is a column body, wherein the column body comprises a hollow space for receiving the boron carbide support material, and wherein the hollow space of the column body is at least twice as long, preferably 3, 4, 5, or 6 times as long as it is wide.

12. Method for producing a column body according to Claim 11, **characterized in that** a column body according to Claim 11 is loaded with a boron carbide support material.

13. Use of a boron carbide support material, a matrix which comprises boron carbide or a matrix according to Claim 11 for binding polypeptides.

14. Kit for purifying and/or isolating polypeptides, **characterized in that** it includes a matrix comprising boron carbide or a matrix according to Claim 11.

15. Kit according to Claim 14, **characterized in that** it includes one or more of the following components:
- an acidic binding and/or equilibration buffer and/or
- an alkaline elution buffer.

## Revendications

1. Procédé d'isolation et/ou de purification d'au moins un polypeptide à partir d'un échantillon contenant des polypeptides, **caractérisé en ce que** l'échantillon est mis en contact avec un matériau de support au carbure de bore à une valeur de pH qui permet la liaison du polypeptide au matériau de support au carbure de bore

2. Procédé selon la revendication 1, **caractérisé en ce que** l'isolation du polypeptide, au moins au nombre de un, se produit pour retirer celui-ci de l'échantillon.

3. Procédé selon la revendication 1 ou 2, **caractérisé en ce que** le polypeptide, au moins au nombre de un, est élué du matériau de support au carbure de bore

4. Procédé selon l'une des revendications 1 à 3, **caractérisé en ce que** la liaison se produit à une valeur de pH qui est inférieure au pl du polypeptide à lier.

5. Procédé selon une ou plusleurs des revendications 1 à 4, **caractérisé en ce que** la liaison se produit à une valeur de pH acide.

6. Procédé selon une ou plusieurs des revendications 1 à 5, **caractérisé en ce que** le matériau de support au carbure de bore est équilibré à une valeur de pH acide.

7. Procédé selon la revendication 5 ou 6, **caractérisé en ce que** la valeur de pH est ≤ 6, ≤ 5, ≤ 4,5, ≤ 4 ou ≤ 3,5.

8. Procédé selon une ou plusieurs des revendications 1 à 7, **caractérisé en ce que** l'élution d'au moins un polypeptide se fait à une valeur de pH qui est supérieure au pl du polypeptide à éluer.

9. Procédé selon la revendication 8, **caractérisé en ce que** la valeur de pH est ≥ 9, 10 ou ≥ 11.

10. Procédé selon une ou plusieurs des revendications 1 à 9, **caractérisé en ce que** l'échantillon est sélectionné dans le groupe composé des solutions salines, des fluides corporels, du plasma, de l'urine, des lysats cellulaires, du sperme, des larmes, de la sueur, de la salive et du liquide cérébral.

11. Matrice destinée à la liaison de polypeptides, **caractérisée en ce qu**'elle présente du carbure de bore et en ce que la matrice est un corps en colonne, le corps en colonne présentant un espace creux permettant de loger le matériau de support au carbure de bore, l'espace creux du corps en colonne étant au moins deux fois plus long, de préférence 3, 4, 5 ou 6 fois plus long que large.

12. Procédé de fabrication d'un corps en colonne selon la revendication 11, **caractérisé en ce qu'**un corps en colonne selon la revendication 11 est chargé d'un matériau de support au carbure de bore.

13. Utilisation d'un matériau de support au carbure de bore, d'une matrice qui présente du carbure de bore ou d'une matrice selon la revendication 11 pour la liaison de polypeptides,

14. Kit ou ensemble de purification et/ou d'isolation de polypeptides, **caractérisé en ce qu'**il présente une matrice présentant du carbure de bore ou une matrice selon la revendication 11.

15. Kit ou ensemble selon la revendication 14, **caractérisé en ce que** celui-ci présente un ou plusieurs des composants suivants :
- un tampon de liaison et/ou d'équilibrage acide, et/ou
- un tampon d'élution alcalin.
